# EUROPEAN PATENT APPLICATION

(11) **EP 4 545 538 A1**
(43) Date of publication of application: **30.04.2025**
(21) Application number: 23826499.8
(22) Date of filing: 21.06.2023
(51) Int. Cl.: C07F 5/02, A61K 31/69, A61P 29/00, A61P 11/00, A61P 13/12, A61P 3/10, A61P 27/02, A61P 1/04

(54) **CRYSTAL OF BORATE DERIVATIVE, METHOD FOR PREPARING SAME, AND USE THEREOF**

(30) Priority: 24.06.2022 CN 202210728955
(71) Applicant: Reistone Biopharma Company Limited, Shanghai 201210 (CN)
(72) Inventor: SUN, Longwei, Shanghai 201210 (CN); HOU, Yanting, Shanghai 201210 (CN); ZHU, Jiajun, Shanghai 201210 (CN); WANG, Zhongli, Shanghai 201210 (CN); XIE, Lan, Shanghai 201210 (CN); LUO, Zhiyang, Shanghai 201210 (CN)
(74) Representative: Potter Clarkson
(86) International application number: PCT/CN2023/101645
(87) International publication number: WO 2023/246854

(57) **Abstract**

The present disclosure relates to a crystal of a borate derivative, a method for preparing same, and use thereof. Specifically, the present disclosure provides a crystal of a compound of formula I, a method for preparing same, and use thereof.

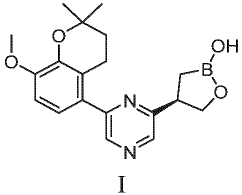

## Description

### FIELD OF THE INVENTION

The present disclosure belongs to the field of medicine, and relates to a crystal of a borate derivative, a method for preparing the same, and a use thereof.

### BACKGROUND OF THE INVENTION

Phosphodiesterases (PDEs) are a class of intracellular enzymes that cleavage phosphodiester bonds on 3',5'-cyclic adenosine monophosphate (cAMP) and 3',5'-cyclic guanosine monophosphate (cGMP) as the second messenger molecules. The cyclic nucleotides cAMP and cGMP act as second messengers in various cellular pathways. Among them, PDE4 is highly specific to cAMP and has four subtypes of PDE4A, PDE4B, PDE4C and PDE4D. PDE4 is involved in promoting activation of monocyte and macrophage, neutrophil infiltration, vascular smooth muscle proliferation, vasodilation and myocardial contraction and other related physiological and pathological processes, and has effects on central nervous system function, cardiovascular function, inflammation/immune system, cell adhesion, and the like. PDE4 plays a major regulating role in the expression of pro-inflammatory and anti-inflammatory mediators, and PDE4 inhibitors can inhibit the release of harmful mediators by inflammatory cells.

Many PDE4 inhibitors have been identified in recent years. For example, roflumilast is approved for severe chronic obstructive pulmonary disease (COPD) to reduce the times of sudden onset or to prevent worsening of COPD symptoms, and apremilast is approved for the treatment of adults with active psoriasis arthritis. Although PDE4 inhibitors show good pharmacological activity, these PDE inhibitors have side effects, such as induced gastrointestinal symptoms such as emesis and diarrhea. There is still a need to develop selective PDE4 inhibitors, especially selective PDE4 inhibitors with affinity to PDE4B and PDE4D.

Crisaborole, a boron (B)-containing drug, was approved by FDA on December 14, 2016, as a topical therapeutic drug for mild to moderate atopic dermatitis. The boron atom facilitates skin penetration and binds to the bimetallic center of Phosphodiesterase 4 (PDE4). In addition, other small molecular boron-containing PDE inhibitors have been reported, such as CN102014927A and WO2020070651. However, the compounds of the present disclosure are not disclosed in any literature, and such compounds exhibit specific PDE4 inhibition effects.

PCT/CN2021/141010 provides a novel PDE4 inhibitor named (R) -4- (6-(8-methoxy-2,2-dimethylbenzodihydropyran-5-yl) pyrazin-2-yl) -1,2-oxaborocyclopentan-2-ol, with the structure shown in formula I,

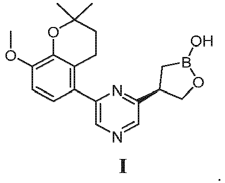

The crystal form of a pharmaceutically active ingredient often affects the chemical stability of a drug, and different crystallization and storage conditions may lead to changes in the crystal structure of a compound, sometimes accompanied by production of other crystal forms. In general, an amorphous drug product which does not have a regular crystal structure often has other defects such as poor product stability, excessively fine crystal, difficult filtration, easy agglomeration, poor flowability and the like. The polymorphic forms of a drug have different requirements on product storage, production, and amplification. Therefore, it is necessary to conduct in-depth research on a crystal form of the aforementioned compound so as to improve various properties of the aforementioned compound.

### SUMMARY OF THE INVENTION

The present disclosure provides crystal form A of the compound of formula I, characterized in that the crystal form A has an X-ray powder diffraction spectrum represented by diffraction angle 2*θ* angle having characteristic peaks at 7.537°, 15.219°, 21.798°, and 22.911°,

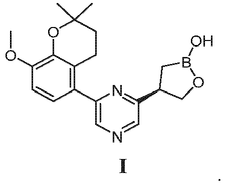

In some embodiments, the crystal form A of the compound of formula I has an X-ray powder diffraction spectrum represented by diffraction angle 2*θ* angle having characteristic peaks at 7.537°, 11.366°, 14.297°, 15.219°, 16.190°, 17.623°, 19.134°, 21.798°, 22.911°, and 24.735°.

In some embodiments, the crystal form A of the compound of formula I has an X-ray powder diffraction spectrum represented by diffraction angle 2*θ* angle having characteristic peaks at 7.537°, 11.366°, 14.018°, 14.297°, 15.219°, 16.190°, 17.623°, 19.134°, 21.062°, 21.798°, 22.911°, 24.735°, 25.123° and 26.884°.

In some embodiments, the crystal form A of the compound of formula I has an X-ray powder diffraction spectrum represented by diffraction angle 2*θ* angle having characteristic peaks at 7.537°, 11.366°, 14.018°, 14.297°, 15.219°, 16.190°, 17.623°, 18.412°, 19.134°, 20.010°, 21.062°, 21.798°, 22.911°, 23.889°, 24.735°, 25.123°, 26.884°, 28.343°, 29.645°, 33.499° and 36.362°.

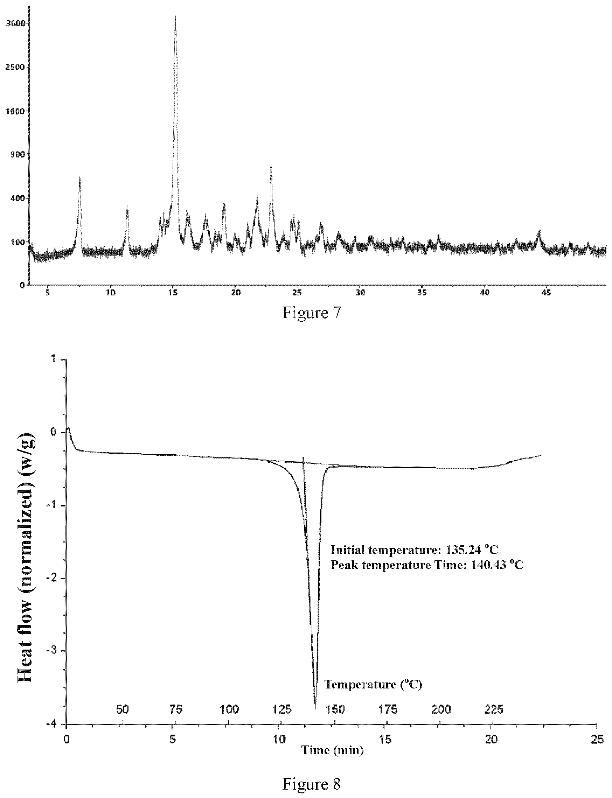

In some embodiments, the crystal form A of the compound of formula I has an X-ray powder diffraction spectrum represented by diffraction angle 2*θ* as shown in Figure 7.

In some embodiments, the crystal form A of the compound of formula I of the present disclosure has the error range of 2*θ* angle of ±0.2°.

The present disclosure also provides a method for preparing the crystal form A of the compound of formula (I) as described above, being method I or method II:
method I comprises the following steps of:
   (a1) mixing the compound of formula I with a solvent (1);
   (b1) crystallizing under stirring; 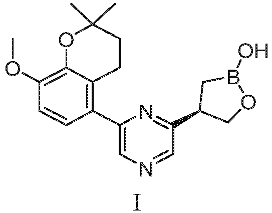
wherein, the solvent (1) is one or more selected from the group consisting of water, nitromethane, N,N-dimethylformamide (DMF), acetonitrile, methanol, ethanol, acetone, isopropanol (IPA), methyl ethyl ketone (MEK), tetrahydrofuran (THF), 1,2-dimethoxyethane, chloroform, methyl tert-butyl ether (MTBE), 1,2-xylene, toluene, dioxane, and hexane; and
method II comprises the following steps of:
   (a2) mixing the compound of formula I with a solvent (2);
   (b2) crystallizing under volatilization;
wherein, the solvent (2) is one or more selected from the group consisting of acetone, acetonitrile, dichloromethane (DCM), dioxane, ethanol, ethyl acetate, heptane, isopropanol, methanol, methyl ethyl ketone, methyl tert-butyl ether, toluene, and water.

In some embodiments, the solvent (1) is water, nitromethane, N,N-dimethylformamide, acetonitrile, ethanol, acetone, isopropanol, methyl ethyl ketone, tetrahydrofuran, 1,2-dimethoxyethane, chloroform, methyl tert-butyl ether, 1,2-xylene, toluene, dioxane, hexane, or a mixed solvent of water and methanol.

In some embodiments, when the solvent (1) is a mixed solvent of water and methanol, the molar ratio of the water to the methanol is 0.37 to 0.90, and may be 0.37, 0.40, 0.47, 0.50, 0.57, 0.60, 0.66, 0.70, 0.74, 0.80, 0.82, and 0.90 in non-limiting embodiments.

In some embodiments, the temperature of the crystallization under stirring is 10°C to 60°C, and may be 10°C, 20°C, 25°C, 30°C, 35°C, 40°C, 45°C, 50°C, 55°C, and 60°C in non-limiting embodiments.

In some embodiments, , the temperature of the crystallization under stirring is room temperature.

In some embodiments, when the temperature of the crystallization under stirring is 10°C to 40°C (e.g., room temperature), the solvent (1) is water, nitromethane, acetonitrile, ethanol, acetone, isopropanol, methyl ethyl ketone, tetrahydrofuran, 1,2-dimethoxyethane, chloroform, methyl tert-butyl ether, 1,2-xylene, toluene, dioxane, hexane, or a mixed solvent of water and methanol.

In some embodiments, when the temperature of the crystallization under stirring is 41°C to 60°C (e.g., 50°C), the solvent (1) is water, N,N-dimethylformamide, acetonitrile, acetone, methyl ethyl ketone, 1,2-dimethoxyethane, chloroform, methyl tert-butyl ether, 1,2-xylene, toluene, hexane, or a mixed solvent of water and methanol.

In some embodiments, the crystallization under stirring is crystallization by pulping.

In some embodiments, the solvent (2) is acetone, acetonitrile, dichloromethane, dioxane, ethyl acetate, heptane, isopropanol, methyl ethyl ketone, methyl tert-butyl ether, toluene, water, or any group of following mixed solvents: acetone and acetonitrile, acetone and dichloromethane, acetone and dioxane, acetone and ethanol, acetone and ethyl acetate, acetone and heptane, acetone and isopropanol, acetone and methanol, acetone and methyl ethyl ketone, acetone and methyl tert-butyl ether, acetone and tetrahydrofuran, acetone and toluene, acetone and water, acetonitrile and dichloromethane, acetonitrile and dioxane, acetonitrile and ethanol, acetonitrile and ethyl acetate, acetonitrile and isopropanol, acetonitrile and methyl ethyl ketone, acetonitrile and methyl tert-butyl ether, acetonitrile and tetrahydrofuran, acetonitrile and toluene, dichloromethane and dioxane, dichloromethane and ethanol, dichloromethane and ethyl acetate, dichloromethane and heptane, dichloromethane and isopropanol, dichloromethane and methyl ethyl ketone, dichloromethane and methyl tert-butyl ether, dichloromethane and tetrahydrofuran, dichloromethane and toluene, dioxane and methyl ethyl ketone, dioxane and tetrahydrofuran, dioxane and toluene, ethanol and ethyl acetate, ethanol and methanol, ethanol and tetrahydrofuran, ethanol and toluene, ethanol and water, ethyl acetate and heptane, ethyl acetate and isopropanol, ethyl acetate and methanol, ethyl acetate and methyl ethyl ketone, ethyl acetate and methyl tert-butyl ether, ethyl acetate and toluene, heptane and methyl ethyl ketone, heptane and methyl tert-butyl ether, heptane and tetrahydrofuran, heptane and toluene, methanol and methyl ethyl ketone, methanol and methyl tert-butyl ether, methanol and toluene, methanol and water, methyl ethyl ketone and methyl tert-butyl ether, methyl ethyl ketone and tetrahydrofuran, methyl ethyl ketone and toluene, methyl tert-butyl ether and tetrahydrofuran, methyl tert-butyl ether and toluene, tetrahydrofuran and toluene, tetrahydrofuran and water.

In some embodiments, when the solvent (2) is a mixed solvent, the volume ratio of the two is 1:3 to 3:1, preferably 1:1.

In some embodiments, the temperature of the crystallization by volatilization is 10°C to 40°C, and may be 10°C, 20°C, 25°C, 30°C, 35°C, and 40°C in non-limiting embodiments.

In some embodiments, the temperature of the crystallization by volatilization is room temperature.

In some embodiments, the volume (µl) of the solvents (1) or (2) may be 1 to 200 times the mass (mg) of the compound of formula I, and may be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200 times or any value between two numbers in non-limiting embodiments.

In some embodiments, it further includes a step of filtration, washing, or drying after the crystallization under stirring or the crystallization under volatilization,.

The present disclosure also provides a pharmaceutical composition comprising the above crystal form A or the crystal form A prepared by the above preparation method, and optionally a pharmaceutically acceptable excipient.

The present disclosure also provides a method for preparing the pharmaceutical composition described above, comprising a step of mixing the above crystal form A or the crystal form A prepared by the above method with a pharmaceutically acceptable excipient.

The present disclosure also provides a use of the above crystal form A, or the crystal form A prepared by the above preparation method, or the above pharmaceutical composition, in the preparation of medicaments for the prevention and/or treatment of PDE-related diseases. In some embodiments, the PDE-related disease is asthma, obstructive pulmonary disease, septicaemia, nephritis, diabetes, allergic rhinitis, allergic conjunctivitis, ulcerative enteritis or rheumatism.

The present disclosure also provides a use of the above crystal form A, or the crystal form A prepared by the above preparation method, or the above pharmaceutical composition, in the preparation of medicaments for the prevention and/or treatment of asthma, obstructive pulmonary disease, septicaemia, nephritis, diabetes, allergic rhinitis, allergic conjunctivitis, ulcerative enteritis or rheumatism.

The term "2*θ* or 2*θ* angle" in the present disclosure refers to a diffraction angle, θ being the Bragg angle, and the unit of which is ° or degree. The error range of 2θ of each characteristic peak is ±0.2 (including the case in which a number is rounded to one decimal), and may be -0.20, -0.19, -0.18, -0.17, -0.16, -0.15, -0.14, -0.13, -0.12, -0.11, -0.10, -0.09, -0.08, -0.07, -0.06, -0.05, -0.04, -0.03, -0.02, -0.01, 0.00, 0.01, 0.02, 0.03, 0.04, 0.05, 0.06, 0.07, 0.08, 0.09, 0.10, 0.11, 0.12, 0.13, 0.14, 0.15, 0.16, 0.17, 0.18, 0.19, or 0.20.

The '9103 Guidelines for Hygroscopicity of Drugs' in Volume IV of the 2015 edition of Chinese Pharmacopoeia describes the characteristics of hygroscopicity and defines the weight gain by hygroscopicity as follows,
deliquescent: absorbing sufficient moisture to form a liquid;
extremely hygroscopic: the weight gain by hygroscopicity being no less than 15%;
hygroscopic: the weight gain by hygroscopicity being less than 15% but not less than 2%;
slightly hygroscopic: the weight gain by hygroscopicity being less than 2% but not less than 0.2%;
no or almost no hygroscopic: the weight gain by hygroscopicity being less than 0.2%.

The term "differential scanning calorimetry or DSC" in the present disclosure means to measure the temperature difference and heat flow difference between the sample and the reference during the temperature of the sample is raising or constant, to characterize all physical and chemical changes associated with the thermal effect to obtain phase change information of the sample.

The temperature for drying in the present disclosure generally ranges from 25°C to 100°C, preferably from 40°C to 70°C. The drying may be performed under normal pressure, or under reduced pressure, with a pressure of less than -0.08 MPa.

The "room temperature" in the present disclosure is 25^{°}C ± 5^{°}C.

The term "excipient" in the present disclosure includes, but is not limited to, any adjuvant, carrier, glidant, sweetener, diluent, preservative, dye/colorant, flavor enhancer, surfactant, wetting agent, dispersant, suspending agent, stabilizer, isotonic agent or emulsifier that has been approved by the U.S. Food and Drug Administration for use in humans or livestock animals.

The term "pulping" in the present disclosure refers to a method of purification which utilizes the property that the solubility of a compound is poor in a solvent, while the solubility of impurities is good in the solvent. Pulping purification can remove color, change crystal form or remove small amounts of impurities.

The starting material used in the method for preparing the crystal form of the present disclosure can be a compound in any form including, but not limited to, amorphous form, arbitrary crystal form, hydrate, solvate and the like.

In the present disclosure, the values, such as those related to the determination and calculation of substance contents, inevitably have a degree of error. In general, ±10% belongs to a reasonable range of error. There is a degree of error depending on the context in which it is used, and the error is no more than ±10% and may be ±9%, ±8%, ±7%, ±6%, ±5%, ±4%, ±3%, ±2% or ±1%, preferably ±5%.

The compound of formula I of (*R*) -4- (6-(8-methoxy-2,2-dimethylbenzodihydropyran-5-yl) pyrazin-2-yl) -1,2-oxaborocyclopentan-2-ol (amorphous, hereinafter referred to as Compound I) in the present disclosure is prepared according to the method in PCT/CN2021/141010, the related contents of which are incorporated herein by reference for illustration.

### DESCRIPTION OF THE DRAWINGS

Figure 1 shows an XRPD spectrum of amorphous Compound I.
Figure 2 shows a graph of clinical score comparison of the compound of each group on a disease model in Test Example 4.
Figure 3 shows a graph of clinical score comparison of the compound of each group on erythema disease model in Test Example 4.
Figure 4 shows a graph of clinical score comparison of the compound of each group on psoriasis disease model in Test Example 4.
Figure 5 shows a graph of comparison of the inhibitory effect of the compound of each group on the increase in skin thickness in Test Example 4.
Figure 6 shows a graph of comparison of the effect of the compound of each group on the ratio of spleen weight to body weight in Test Example 4.
Figure 7 shows an XRPD spectrum of the crystal form A.
Figure 8 shows a DSC spectrum of the crystal form A.
Figure 9 shows a TGA spectrum of the crystal form A.
Figure 10 shows a DVS spectrum of the crystal form A.

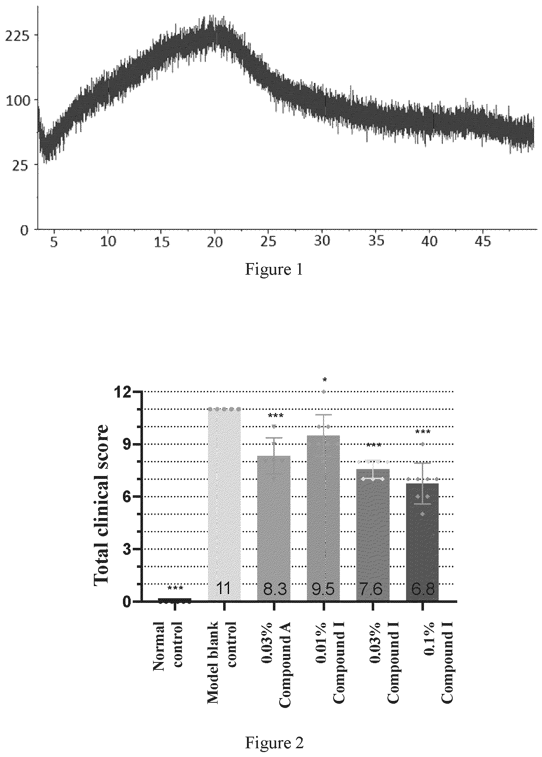

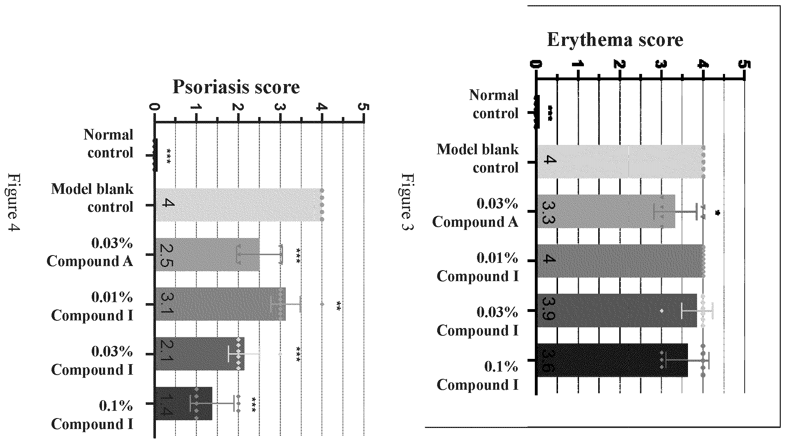

In Figures 1-6, * represents p<0.05 in comparison with the model group, ** represents p<0.01 in comparison with the model group, and *** represents p<0.001 in comparison with the model group. The symbols ▼, ●, ◆ and # are only used to distinguish one group from another and have no real meaning.

### DETAILED DESCRIPTION OF THE INVENTION

The present disclosure will be illustrated in more detail through examples below. The examples or experimental examples of the present disclosure are merely intended to describe the technical solutions of the present disclosure, and should not be considered as limiting the spirit and scope of the present disclosure.

The abbreviations used in the present disclosure are explained as follows:
- XRPD: X-ray powder diffraction
- DSC: Differential scanning calorimetry
- TGA: Thermogravimetric analysis
- DVS: Dynamic vapor sorption
- ¹H-NMR: Liquid nuclear magnetic resonance hydrogen spectroscopy
- DMF: *N,N*-dimethylformamide
- MEK: Methyl ethyl ketone
- MTBE: Methyl tert-butyl ether
- THF: Tetrahydrofuran
- IPA: Isopropanol
- DCM: Dichloromethane
- ACN: Acetonitrile

Test conditions for the instruments used in experiments of the present disclosure:
1. X-ray Powder Diffraction, XRPD
   Instrument Model: Malver Panalytical Aeris X-ray powder diffractometer
   Ray: monochromatic Cu-Kα ray (λ=1.54188)
   Scanning mode: θ/2θ, Scanning range (2θ range) : 3.5-50°
   Voltage: 40 kV, Electric current: 15 mA.
2. Differential Scanning Calorimetry, DSC
   Instrument Model: TA DSC250
   Purge gas: Nitrogen; Nitrogen purge rate: 50 mL/min
   Heating rate: 10°C/min
   Temperature range: 25°C-300°C.
3. Thermogravimetric Analysis, TGA
   Instrument Model: TA TGA550
   Purge gas: Nitrogen; Nitrogen purge rate: 20 ml/min
   Heating rate: 10°C/min
   Temperature range: 30°C-350°C.
4. Dynamic vapor sorption (DVS)
   The detection is performed using SMS Intrinsic PLUS at 25°C, with humidity change of 50% -0% -90% with a step size of 10%, in which the criteria for judging is that the mass change dM/dT for each gradient is less than 0.0002%, TMAX is 360 min, and two cycles are performed.
5. High performance liquid chromatography (HPLC) described in the stability test of the crystal form A of the present disclosure is determined on Agilent 1260 Infinity II under the following HPLC conditions: chromatographic column of Agilen XDB C18 5 µm 4.6 × 250 mm; mobile phase of A - 0.05% TFA (in water), and B - ACN; flow rate of 1.0 ml/min; wavelength of 220 nm.
6. The structures of the compounds are identified by nuclear magnetic resonance (NMR) and/or mass spectrometry (MS). NMR shift (δ) is given in units of 10⁻⁶ (ppm). NMR is determined on a Bruker AVANCE-400 nuclear magnetic spectrometer. The solvents for determination are deuterated dimethyl sulfoxide (DMSO-*d₆*), deuterated chloroform (CDCl₃), and deuterated methanol (Methanol-*d₄*), and the internal standard is tetramethylsilane (TMS).
7. HPLC in Preparation Example of Compound I is determined on an Agilent1100 high pressure liquid chromatograph with a GAS15B DAD UV detector and Water Vbridge C18 150 × 4.6 mm 5 µm column.
8. MS is determined on an Agilent6120 triple quadrupole mass spectrometer with a G1315D DAD detector and a Waters Xbridge C18 4.6 × 50 mm, 5 µM column, scanning in a positive/negative ion mode with a mass scanning range from 80 to 1200.
9. Preparative HPLC conditions: water; column of Sunfire (Prep C18 OBD 19 × 250 mm 10 µm).
10. Chiral column resolution conditions: column of Chiralpak IG 5 µm 30 × 250 mm; mobile phase of Hex : EtOH = 35 : 65, 15 mL/min; temperature of 30°C; wavelength of 254 nm.
11. Yantai Huanghai HSGF254 silica gel plates are used for the thin-layer chromatography. For the gel plates used, the specification is 0.2 mm ± 0.03 mm for thin-layer chromatography (TLC), and 0.4 mm - 0.5 mm for separation and purification of products by thin-layer chromatography.
12. Combiflash Rf150 (TELEDYNE ISCO) or Isolara one (Biotage) is used as the rapid column purification system.
13. Yantai Huanghai 200~300 mesh silica gel or 300~400 mesh silica gel is generally used as a carrier for normal-phase column chromatography, or Changzhou Santai prefilled ultra-pure normal-phase silica gel columns (40-63 µm, 60 g, 24 g, 40 g, 120 g or other specifications) are used.

The known starting materials of the present disclosure can be synthesized by or according to the methods known in the art, or can be purchased from Shanghai Titan Technology, ABCR GmbH & Co. KG, Acros Organics, Aldrich Chemical Company, Accela ChemBio Inc, Bide Pharm and other companies.

Unless specifically stated in the examples, all reactions can be carried out under nitrogen atmosphere.

Nitrogen atmosphere means that a reaction flask is connected to a nitrogen balloon with a volume of about 1 L.

Hydrogen atmosphere means that a reaction flask is connected to a hydrogen balloon with a volume of about 1 L.

Hydrogen is produced by a hydrogen generator of the QPH-1L type from Shanghai Quan Pu Scientific Instruments.

The nitrogen atmosphere or hydrogen atmosphere is generally generated by vacuuming and filling with nitrogen or hydrogen, 3 times repeatedly.

Unless specifically stated in the examples, a solution refers to an aqueous solution.

Unless specifically stated in the examples, the reaction temperature is room temperature, which is 20°C to 30°C.

Reaction processes in the examples are monitored by thin-layer chromatography (TLC). The developing agents used for the reactions, the eluent systems of the column chromatography and the developing systems of the thin-layer chromatography used for purifying the compounds, and the volume ratios of the solvents are adjusted depending on the polarity of the compounds. A small amount of basic or acidic reagents such as triethylamine and acetic acid can also be added for adjustment.

### Example 1: Preparation of Compound I

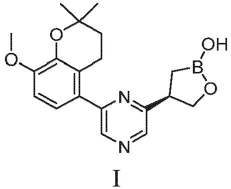

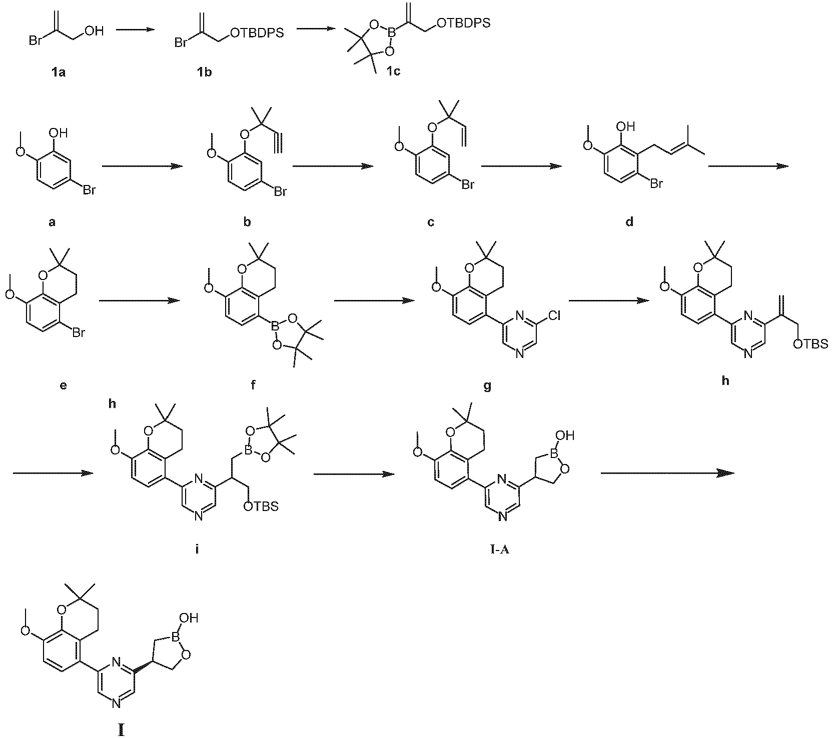

Step 1): Compound **1a** (2.0 g, 14.6 mmol) and triethylamine (1.8 g, 17.8 mmol) were dissolved in N,N-dimethylformamide (30 mL) and the solution was cooled to 0°C. Tert-butyldiphenylchlorosilane (4.0 g, 14.6 mmol) was added dropwise to the reaction system under nitrogen atmosphere. The reaction system was warmed to room temperature and continually stirred until completion of the reaction was detected by TLC. The reaction solution was poured into water and extracted with ethyl acetate (100 mL × 3). The organic phase was washed with water (50 mL × 2), dried over anhydrous sodium sulfate and concentrated. The residues were purified by silica gel column chromatography (ethyl acetate/petroleum ether) to obtain Compound **1b** (5.1 g), which was directly used in the next reaction.

Step 2): A mixture of Compound **1b** (5.1 g, 13.6 mmol), bis(pinacolato)diboron (4.2 g, 16.3 mmol), [1,1'-bis(diphenylphosphino)ferrocene]palladium dichloride (512 mg, 0.7 mmol) and potassium acetate (2.0 g, 20.4 mmol) in dioxane (100 mL) was warmed to 80°C and stirred overnight under nitrogen atmosphere. The reaction solution was poured into water and extracted with ethyl acetate (100 mL × 2). The organic phase was washed with water (30 mL × 2), dried over anhydrous sodium sulfate and concentrated. The residues were purified by silica gel column chromatography (petroleum ether/ethyl acetate) to obtain Compound **1c** (2.0 g). LCMS: m/z 423.2 (M+H)⁺.

Step 3): Compound **a** (100 g, 492 mmol), DMF (1000 mL), potassium iodide (92.6 g, 837 mmol), cuprous iodide (3.13 g, 9.85 mmol) and potassium carbonate (136 g, 985 mmol) were added to a 2000 mL single-neck flask successively at room temperature. 3-Chloro-3-methyl-1-butyne (100 mL, 886 mmol) was added dropwise thereto under nitrogen atmosphere. The reaction mixture was stirred for 16 hours at 70°C. The resulting mixture was cooled to room temperature. Water (1000 mL) was added, and the mixture was extracted with petroleum ether (1000 mL × 3). The organic phase was dried over anhydrous sodium sulfate, and filtered. The filtrate was concentrated under reduced pressure, and the residues were purified by column chromatography (petroleum ether/ethyl acetate) to obtain Compound **b** (50 g).

*¹H* NMR (400 MHz, CDCl₃) δ 7.57 (d, *J =* 2.4 Hz, 1H), 7.15 (dd, *J =* 8.8, 2.4 Hz, 1H), 6.76 (d, *J =* 8.8 Hz, 1H), 3.79 (s, 3H), 2.58 (s, 1H), 1.65 (s, 6H).

Step 4): Compound **b** (20.0 g, 74.4 mmol), n-hexane (200 mL) and palladium calcium carbonate (1.95 g, 18.8 mmol) were added to a 500 mL single-neck flask successively at room temperature. The reaction mixture was stirred for 16 hours at room temperature under hydrogen atmosphere. The resulting mixture was filtered and concentrated under reduced pressure to obtain Compound c (19 g).

*¹H* NMR (400 MHz, CDCl₃) δ 7.15 (d, *J =* 2.4 Hz, 1H), 7.09 (dd, *J =* 8.8, 2.4 Hz, 1H), 6.73 (d, *J =* 8.8 Hz, 1H), 6.12 (dd, *J =* 17.6, 10.8 Hz, 1H), 5.14 (dd, *J =* 20.0, 9.2 Hz, 2H), 3.79 (s, 3H), 1.46 (s, 6H).

Step 5): Compound c (10.0 g, 36.9 mmol) and diethylaniline (10 mL, 62.5 mmol) were added to a 50 mL single-neck flask successively at room temperature. The reaction mixture was stirred for 1 hours at 210°C. The resulting mixture was cooled to room temperature, adjusted to neutral pH with 1 M HCl, extracted with ethyl acetate (200 mL × 3), and washed with water (200 mL × 3). The organic phase was concentrated under reduced pressure to obtain Compound **d** (9.1 g).

*¹H* NMR (400 MHz, DMSO-*d*₆) δ 8.97 (s, 1H), 6.97 (d, *J =* 8.8 Hz, 1H), 6.77 (d, *J* = 8.8 Hz, 1H), 5.14-5.01 (m, 1H), 3.78 (s, 3H), 3.40 (d, *J =* 6.8 Hz, 2H), 1.74 (s, 3H), 1.63 (s, 3H).

Step 6): Compound **d** (5.00 g, 18.5 mmol), toluene (25 mL) and Amberlyst^{®} 15 (5.00 g, 15.9 mmol) were added to a 100 mL single-neck flask successively at room temperature. The reaction mixture was stirred for 2 hours at 100°C under nitrogen atmosphere. The resulting mixture was cooled to room temperature and filtered. The filtrate was concentrated under reduced pressure to obtain Compound **e** (3.89 g).

*¹H* NMR (400 MHz, DMSO-*d*₆) δ 7.04 (d, *J =* 8.8 Hz, 1H), 6.75 (d, *J =* 8.8 Hz, 1H), 3.71 (s, 3H), 2.63 (t, *J =* 6.8 Hz, 2H), 1.78 (t, *J =* 6.8 Hz, 2H), 1.26 (s, 6H).

Step 7): Compound **e** (28.4 g, 105 mmol), dioxane (300 mL), bis(pinacolato)diboron (31.9 g, 126 mmol), potassium acetate (20.6 g, 209 mmol) and [1,1'-bis(diphenylphosphino)ferrocene]palladium dichloride (7.66 g, 10.5 mmol) were added to a 500 mL single-neck flask successively at room temperature. The reaction mixture was stirred for 16 hours at 105^{°}C under nitrogen atmosphere. The resulting mixture was cooled to room temperature and filtered. The filtrate was concentrated under reduced pressure, and the residues were purified by column chromatography (petroleum ether/ethyl acetate) to obtain Compound f (28.4 g).

LCMS: m/z 319 (M+H) ⁺.

Step 8): Compound **f** (6.20 g, 19.5 mmol), 1,4-dioxane (80 mL), water (16 mL), 2,6-dichloropyrazine (2.90 g, 39.0 mmol), potassium carbonate (5.39 g, 39.0 mmol) and [1,1'-bis(diphenylphosphino)ferrocene]palladium dichloride (1.43 g, 1.95 mmol) were added to a 250 mL single-neck flask successively at room temperature. The reaction mixture was stirred for 16 hours at 110°C under nitrogen atmosphere. The resulting mixture was cooled to room temperature and filtered. The filtrate was concentrated under reduced pressure, and the residues were purified by column chromatography (petroleum ether/ethyl acetate) to obtain Compound **g** (5.7 g).

LCMS: m/z 305 (M+H) ⁺.

Step 9): Compound **g** (35.0 g, 115 mmol), Compound 1c (44.5 g, 149 mmol), potassium carbonate (23.8 g, 172 mmol), potassium acetate (16.9 g, 172 mmol) and [1,1'-bis(diphenylphosphino)ferrocene]palladium dichloride (8.41 g, 11.5 mmol) were added to a mixed solvent of 1,4-dioxane (250 mL) and water (5 mL) in a 500 mL single-neck flask at room temperature. The reaction mixture was stirred until dissolution, purged with nitrogen for 3 times, and stirred for 16 hours at 110^{°}C. The resulting mixture was cooled to room temperature, extracted with ethyl acetate (300 mL × 3), and dried over anhydrous sodium sulfate. The organic phase was filtered and concentrated under reduced pressure, and the residues were purified by column chromatography (petroleum ether/ethyl acetate) to obtain Compound **h** (37 g).

LCMS: m/z 441.1 (M+H) ⁺.

Step 10): 1,2-bis(diphenylphosphino)ethane (1.81 g, 4.54 mmol), (1,5-cyclooctadienyl)methoxyiridium (I) dimer (1.50 g, 2.27 mmol) and anhydrous 1,2-dichloroethane (100 mL) were added to a 250 mL three-neck flask at room temperature. The mixture was stirred for 10 minutes at room temperature, and Compound **h** (10.0 g, 22.7 mmol) was added. The mixture was warmed to 70°C, and pinacolborane (20.3 g, 159 mmol) was added dropwise. The reaction was carried out for 4 hours at 95°C. The resulting mixture was cooled to 0°C, and quenched by adding methanol (50 mL) dropwise. The resulting mixture was concentrated, and the residues were purified by column chromatography (petroleum ether/ethyl acetate) to obtain Compound i (1.4 g).

LCMS: m/z 569.3 (M+H) ⁺.

Step 11): Compound i (9.00 g, 15.8 mmol) and tetrahydrofuran (30 mL) were added to a 50 mL single-neck flask at room temperature. The mixture was cooled to 0°C, and 2 M hydrochloric acid (50 mL) was added. The reaction mixture was stirred for 16 hours at 50°C. The resulting mixture was concentrated under reduced pressure, extracted with ethyl acetate (300 mL × 3), dried over anhydrous sodium sulfate, and filtered. The filtrate was concentrated under reduced pressure, and the residues were purified by reversed-phase column chromatography (acetonitrile/water/trifluoroacetic acid) to obtain Compound **I-A** (3.82 g).

The product was subjected to chiral resolution (column: Chiralpak IG 5 µm 30 * 250 mm; mobile phase: Hex : EtOH = 35: 65; flow rate: 15 mL/min; temperature: 30^{°}C; wavelength: 254 nm) to obtain Compound **I** (shorter retention time).

### Compound I

Compound **I** was determined by XRPD, and the result showed that the product was amorphous. The XRPD spectrum is shown in Figure 1.

LCMS: m/z 355.0 (M+H) ⁺.

*¹H* NMR (400 MHz, DMSO-*d*₆) δ 8.62 (s, 1H), 8.48 (s, 1H), 6.98 (d, *J =* 8.4 Hz, 1H), 6.90 (d, *J =* 8.4 Hz, 1H), 4.29 (dd, *J =* 8.8, 7.6 Hz, 1H), 3.96 (dd, *J =* 8.8, 6.8 Hz, 1H), 3.77 (s, 3H), 3.74-3.62 (m, 1H), 2.93-2.67 (m, 2H), 1.68 (t, *J* = 6.8 Hz, 2H), 1.38-1.24 (m, 7H), 1.21-1.08 (m, 1H).

### Example 2: Preparation of crystal form A of Compound I

10 mg of Compound I was added to 0.2 ml of the solvent as shown in Table 1 below. The compound was kept in a slurry state in the solvent, and stirred for 7 days at room temperature (25 °C) or 50°C. The solvent was quickly removed using a filter paper, and the resulting solid was determined as crystal form A by XRPD.

**Table 1. Solvents selected for preparation of crystal form A through crystallization under stirring and the detection results by XRPD**

| Solvent | Crystal form (stirring at room temperatu re) | Crystal form (stirring at 50 °C) | Solvent | Crystal form (stirring at room temperatu re) | Crystal form (stirring at -50 °C) | Solvent (molar ratio of water in the mixture) | Crystal form (stirring at room temperatur e) | Crystal form (stirring at 50 °C) |
|---|---|---|---|---|---|---|---|---|
| Water | A | A | Tetrahy drofuran | A | N/A | 0.37 (water/met hanol) | A | N/A |
| Nitrometh ane | A | N/A | 1,2-met hoxyeth ane | A | A | 0.47 (water/met hanol) | A | A |
| N,N-dime thylforma mide | N/A | A | Chlorof orm | A | A | 0.57 (water/met hanol) | A | A |
| Acetonitri le | A | A | Methyl tert-buty l ether | A | A | 0.66 (water/met hanol) | A | A |
| Ethanol | A | N/A | 1,2-xyle ne | A | A | 0.74 (water/met hanol) | A | A |
| Acetone | A | A | Toluene | A | A | 0.82 (water/met hanol) | A | A |
| Isopropan ol | A | N/A | Dioxane | A | N/A | 0.90 (water/met hanol) | A | A |
| Methyl ethyl ketone | A | A | Hexane | A | A | | | |

| | | | | | | | | |
|---|---|---|---|---|---|---|---|---|
| Note: N/A means undetected. | | | | | | | | |

### Example 3: Preparation of crystal form A of Compound I

5 mg of Compound I was added to the solvent as shown in Table 2 below. Volatilization was performed at room temperature, and after 7 days, the resulting mixture was placed in a drying oven under vacuum at 50 °C to remove the solvent. The resulting solid was determined as crystal form A by XRPD.

**Table 2. Solvents selected for preparation of crystal form A through crystallization under volatilization and the detection results by XRPD**

| Solvent | Crystal form | Solvent | Result | Solvent | Result | Solvent | Crystal form |
|---|---|---|---|---|---|---|---|
| Acetone 0.30 ml | A | Acetonitrile 0.15 ml/Ethyl acetate 0.15 ml | A | Dioxane 0.15 ml/THF 0.15 ml | A | Heptane 0.30 ml/Toluene 0.30 ml | A |
| Acetone 0.15 ml/Acetonitrile 0.15 ml | A | Acetonitrile 0.15 ml/Isopropanol 0.15 ml | A | Dioxane 0.15 ml/Toluene 0.15 ml | A | Isopropanol 0.30 ml | A |
| Acetone 0.15 ml/DCM 0.15 ml | A | Acetonitrile 0.15 ml/MEK 0.15 ml | A | Ethanol 0.15 ml/Ethyl acetate 0.15 ml | A | Methanol 0.15 ml/MEK 0.15 ml | A |
| Acetone 0.15 ml/Dioxane 0.15 ml | A | Acetonitrile 0.15 ml/MTBE 0.15 ml | A | Ethanol 0.15 ml/Methanol 0.15 ml | A | Methanol 0.15 ml/MTBE 0.15 ml | A |
| Acetone 0.15 ml/Ethanol 0.15 ml | A | Acetonitrile 0.15 ml/THF 0.15 ml | A | Ethanol 0.15 ml/THF 0.15 ml | A | Methanol 0.15 ml/Toluene 0.15 ml | A |
| Acetone 0.15 ml/Ethyl acetate 0.15 ml | A | Acetonitrile 0.15 ml/Toluene 0.15 ml | A | Ethanol 0.15 ml/Toluene 0.15 ml | A | Methanol 0.15 ml/Water 0.15 ml | A |
| Acetone 0.30 ml/Heptane 0.30 ml | A | DCM 0.30 ml | A | Ethanol 0.15 ml/Water 0.15 ml | A | MEK 0.30 ml | A |
| Acetone 0.15 ml/Isopropanol 0.15 ml | A | DCM 0.15 ml/Dioxane 0.15 ml | A | Ethyl acetate 0.30 ml | A | MEK 0.15 ml/MTBE 0.15 ml | A |
| Acetone 0.15 ml/Methanol 0.15 ml | A | DCM 0.15 ml/Ethanol 0.15 ml | A | Ethyl acetate 0.30 ml/Heptane 0.30 ml | A | MEK 0.15 ml/THF 0.15 ml | A |
| Acetone 0.15 ml/MEK 0.15 ml | A | DCM 0.15 ml/Ethyl acetate 0.15 ml | A | Ethyl acetate 0.15 ml/Isopropanol 0.15 ml | A | MEK 0.15 ml/Toluene 0.15 ml | A |
| Acetone 0.15 ml/MTBE 0.15 ml | A | DCM 0.30 ml/Heptane 0.30 ml | A | Ethyl acetate 0.15 ml/Methanol 0.15 ml | A | MTBE 0.30 ml | A |
| Acetone 0.15 ml/THF 0.15 ml | A | DCM 0.15 ml/Isopropanol 0.15 ml | A | Ethyl acetate 0.15 ml/MEK 0.15 ml | A | MTBE 0.15 ml/THF 0.15 ml | A |
| Acetone 0.15 ml/Toluene 0.15 ml | A | DCM 0.15 ml/MEK 0.15 ml | A | Ethyl acetate 0.15 ml/MTBE 0.15 ml | A | MTBE 0.15 ml/Toluene 0.15 ml | A |
| Acetone 0.15 ml/Water 0.15 ml | A | DCM 0.15 ml/MTBE 0.15 ml | A | Ethyl acetate 0.15 ml/Toluene 0.15 ml | A | THF 0.15 ml/Toluene 0.15 ml | A |
| Acetonitrile 0.30 ml | A | DCM 0.15 ml/THF 0.15 ml/ | A | Heptane 0.60 ml | A | THF 0.15 ml/Water 0.15 ml | A |
| Acetonitrile 0.15 ml/DCM 0.15 ml | A | DCM 0.15 ml/Toluene 0.15 ml | A | Heptane 0.30 ml/MEK 0.30 ml | A | Toluene 0.30 ml | A |
| Acetonitrile 0.15 ml/Dioxane 0.15 ml | A | Dioxane 0.30 ml | A | Heptane 0.30 ml/MTBE 0.30 ml | A | Water 0.60 ml | A |
| Acetonitrile 0.15 ml/Ethanol 0.15 ml | A | Dioxane 0.15 ml/MEK 0.15 ml | A | Heptane 0.30 ml/ THF 0.30 ml | A | | |

The solid obtained in Examples 3 and 4 was detected by X-ray powder diffraction, and the product was defined as crystal form A. The XRPD spectrum is shown in Figure 7, and the positions of the characteristic peaks are shown in Table 3. The DSC spectrum, as shown in Figure 8, shows an endothermic peak at 140.43 °C. The TGA spectrum, as shown in Figure 8, shows a weight loss of 1.53% from 25 °C to 100 °C.

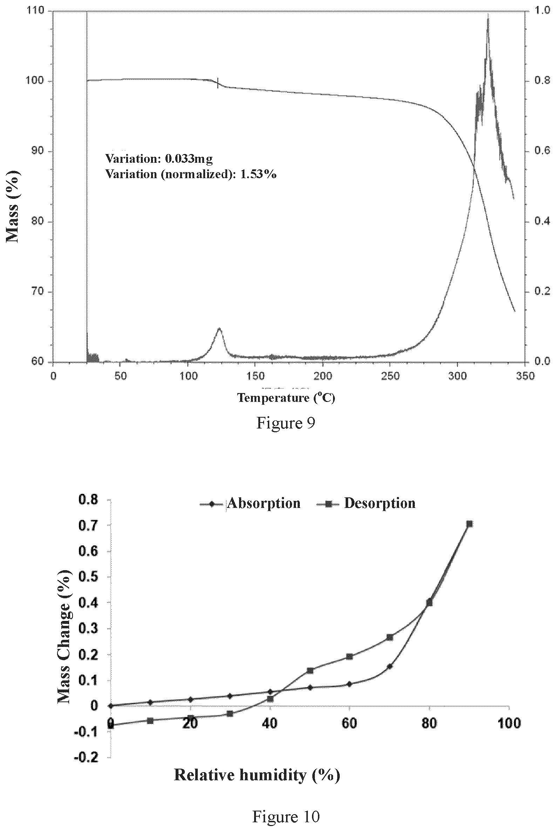

The DVS detection, as shown in Figure 10, shows that the weight gain by hygroscopicity of the sample is approximately 0.08% under normal storage conditions (i.e. 25°C, 60% RH); the weight gain by hygroscopicity is approximately 0.15% under accelerated experimental conditions (i.e. 70% RH); the weight gain by hygroscopicity is approximately 0.70% under extreme conditions (i.e. 90% RH),. During the humidity variation from 0% to 90% RH, the desorption process of the sample coincides with the adsorption process thereof. After DVS detection, the crystal form was redetermined, and XRPD detection shows that the crystal form does not change before and after DVS detection.

**Table 3. Data of XRPD characteristic diffraction peaks of crystal form A**

| Peak No. | 2*θ*[° or degree] | d[Å] | Relative strength |
|---|---|---|---|
| 1 | 7.537 | 11.72982 | 15.06 |
| 2 | 11.366 | 7.78533 | 6.89 |
| 3 | 14.018 | 6.31791 | 3.84 |
| 4 | 14.297 | 6.19531 | 5.05 |
| 5 | 15.219 | 5.82188 | 100.00 |
| 6 | 16.190 | 5.47476 | 4.71 |
| 7 | 17.623 | 5.03270 | 3.77 |
| 8 | 18.412 | 4.81892 | 1.04 |
| 9 | 19.134 | 4.63853 | 6.38 |
| 10 | 20.010 | 4.43749 | 0.88 |
| 11 | 21.062 | 4.21822 | 2.41 |
| 12 | 21.798 | 4.07742 | 8.49 |
| 13 | 22.911 | 3.88175 | 17.83 |
| 14 | 23.889 | 3.72504 | 1.11 |
| 15 | 24.735 | 3.59941 | 4.08 |
| 16 | 25.123 | 3.54480 | 3.14 |
| 17 | 26.884 | 3.31646 | 3.39 |
| 18 | 28.343 | 3.14894 | 1.49 |
| 19 | 29.645 | 3.01356 | 1.09 |
| 20 | 30.915 | 2.89257 | 0.92 |
| 21 | 32.544 | 2.75142 | 0.94 |
| 22 | 33.499 | 2.67513 | 1.26 |
| 23 | 36.362 | 2.47082 | 1.75 |

### Example 4: Stability test I of crystal form A

It is determined whether temperature and humidity have an influence on the stability of crystal form A of the compound of formula I.

The crystal form A was placed in a stabilizing chamber for accelerated stability tests under a series of temperature and humidity combinations. After being placed for different days under various temperature and humidity conditions, the samples were taken out and compared with the initial crystal form A. The content of the compound of formula I was determined by high performance liquid chromatography, and XRPD detection was performed. The test results are shown in Table 4 below.

The results show that the crystal form A remains unchanged and exhibits good stability.

**Table 4. Temperature/humidity conditions for the stability test of crystal form A, contents, and determination results of related substances**

| Temperature (°C) | Humidity (%) | Days (in day) | Crystal form | Purity (%) |
|---|---|---|---|---|
| / | / | 0 | A | 97.99 |
| 55 | 30 | 18 | A | 98.01 |
| 55 | 79 | 20 | A | 97.40 |
| 65 | 11 | 20 | A | 97.74 |
| 65 | 50 | 20 | A | 96.16 |
| 65 | 75 | 20 | A | 95.86 |
| 70 | 11 | 20 | A | 97.33 |
| 75 | 0 | 20 | A | 97.41 |
| 75 | 50 | 7 | A | 96.57 |

### Example 5: Stability test II of crystal form A

It is determined whether light has an influence on the stability of crystal form A of the compound of formula I.

The crystal form A was placed in a light stabilizing chamber, and taken out after reaching ICH strength. The resulting solid was compared with the initial crystal form A. The content of the compound of formula I was determined by high performance liquid chromatography, and XRPD detection was performed. The test results are shown in Table 5 below.

### Example 5. Light stability of crystal form A

| | At 0 | Crystal form A Light-0.5 ICH | Crystal form A Light-1.0 ICH |
|---|---|---|---|
| Purity (%) | 97.99 | 97.76 | 97.15 |
| Crystal form | A | A | A |

### BIOLOGICAL EVALUATIONS

The present disclosure is further described below with reference to the test examples, but these test examples are not intended to limit the scope of the present disclosure.

The structure of Compound A is:

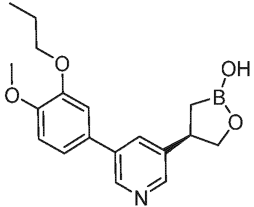

Compound A was prepared by the method disclosed in Example 4 on page 181 of the specification of the patent application WO2020070651A.

### Test Example 1. In vitro PDE4B enzyme activity detection assay

### 1. Experimental materials

| Name | Brand | Cat No./Model |
|---|---|---|
| PDE4B 1 enzyme | BPS | 60041 |
| Trequinsin | TOCRIS | 2337/10 |
| IMAP FP IPP Explorer Kit | Molecular Device | R8124 |
| FAM-cAMP | Molecular Device | R7506 |
| OptiPlate^{™}-384 F black assay plate | PerkinElmer | 6007279 |
| 384 well Echo plate | Labcyte | PP-0200 |
| Frozen PBMC | HemaCare | PB009C-2 |
| RPMI 1640 culture medium | Gibco | 11875093 |
| Roflumilast | Shanghai Bide Pharm | BD150494 |
| Dexamethasone | Sigma-Aldrich | D1756 |
| IL-4 | Aladdin | rp154228 |
| Balb/c female mice | Shanghai Jihui Experimental Animal Breeding Co., Ltd | Balb/c |
| GM-CSF | Acmec | AC13219 |
| Zymosan | Acmec | Z89290 |

### 2. Experimental steps

A stock solution of the compound at a concentration of 10 mM was prepared in a test tube with 90% DMSO (10% water), and it was used to prepare a series of dilutions with a dilution factor of 1:5 and final concentrations starting from 100 µM and lowering to 0.05 nM.

0.2 µl of the solution of the compound was transferred into a 384-well reaction plate, and 0.2 µl of 100% DMSO was transferred into both the negative and positive controls. Then, 10 µl of 2× PDE4B1 enzyme solution (a final concentration of 0.04 nM) was added to the wells, and 10 µl of 1× reaction buffer (50 mM HEPES, pH 7.5, 0.0015% Brij-35) instead of the enzyme solution was added to the wells of the control without enzyme activity. The plate was centrifuged at 1,000 rpm for 1 min and incubated at room temperature for 15 min. Next, 10 µl of 2× FAM-cAMP substrate solution (a substrate final concentration of 0.1 µM) was added to each well of the 384-well reaction plate. The plate was centrifuged at 1,000 rpm for 1 min, and the reaction was carried out at 25°C for 30 min. After completion of the reaction, 60 µL of a reaction stop solution was added to each well of the 384-well reaction plate to terminate the reaction, and the plate was incubated for 60 min in dark under shaking at 600 rpm on a shaker at room temperature.

After completion of the incubation, the RLU data were read and the inhibition rate was calculated. The IC₅₀ value was calculated from the concentration-inhibition fitted curve, wherein the maximum value refers to the reading of the DMSO control and the minimum value refers to the reading of the control without enzyme activity.

The *in vitro* inhibition of PDE4B1 enzyme activity by Compound I of the present disclosure was determined by the above test, and the measured IC₅₀ values are shown in Table 6.

**Table 6**

| No. | PDE4B 1 IC50 (nM) |
|---|---|
| Compound **I** | **0.17** |
| Compound **A** | **0.3** |

### Test Example 2. Inhibitory effect of the compounds on the release of pro-inflammatory cytokines in peripheral blood mononuclear cells (PBMCs)

Frozen PBMCs were thawed and detected by Trypan blue staining for cell viability and number. The thawed PBMCs were washed with RPMI1640 complete medium (RPMI1640 + 10% FBS + 1% PS) and centrifuged, and the supernatant was discarded. The PBMCs were resuspended with RPMI1640 complete medium and the cell density was adjusted to 2×10⁶ cells/mL. 2×10⁵ PBMC cells were plated in a 96-well cell culture plate, and the compounds to be tested were added at different concentrations, in a 1:5 serial dilution of 9 concentrations starting from a maximum compound concentration of 100 µM, each in duplicate wells. LPS was added at a final concentration of 0.1 ng/mL, making a total volume of 200 µL. For the negative and positive controls, only LPS and DMSO were added to the negative control wells, while 1 µg/mL of dexamethasone as a positive control was added to the positive control wells in addition to cells and LPS. The cells were incubated for 24 hours in an incubator at 37°C. After completion of the incubation, 100 µL of cell culture supernatant was collected and detected by ELISA for the TNF-α level. 100 µL of CellTiter-Glo was added to the remaining cells in each well to detect the level of cell viability. The IC₅₀ values of inhibition on TNF-α release by the compounds were calculated.

The *in vitro* inhibition of Compound I of the present disclosure on the release of pro-inflammatory cytokine in PBMCs was determined by the above test, and the measured IC₅₀ values are shown in Table 7.

**Table 7**

| No. | PBMC IC50 (nM) |
|---|---|
| Compound **I** | **0.055** |
| Compound **A** | **1.16** |

### Test Example 3. In vitro inhibition of the compounds on IL-23 secretion in DC cells differentiated from human mononuclear cells

Day 0: Mononuclear cells were isolated from fresh human peripheral blood and purified, and then resuspended in RPMI-1640 complete medium. When differentiation into DC cells took place, IL-4 at a concentration of 50 ng/ml and GM-CSF at 100 ng/ml were added to the medium, and the cells were cultured at a density of 1×10⁶ cells/mL in a culture dish with a diameter of 100 mm for differentiation in an incubator at 37°C with a carbon dioxide concentration of 5%. Day 3: Half the volume of RPMI complete medium was replaced with fresh RPMI complete medium, and the concentrations of IL-4 and GM-CSF were maintained at 50 ng/ml and 100 ng/ml respectively. Day 6: Non-adherent cells (DC cells) in the culture dish were collected and washed with PBS. The washed DC cells were resuspended with RPMI complete medium at a density of 1×10⁶ cells/mL. Then, 1×10⁵ DC cells were added to each well of the 96-well cell culture plate and pre-incubated for 1 hour with different dilutions of the compounds to be tested (or DMSO blank negative control at an equivalent concentration), followed by addition of 200 µg/ml of TLR2 agonist Zymosan to stimulate the DC cells for 24 hours. Day 7: After the DC cells were stimulated by Zymosan for 24 hours, the supernatant in each well of the 96-well plate was collected and detected by ELISA for the concentration of IL-23.

The inhibition of the compounds of the present disclosure on IL-23 secretion in DC cells differentiated from human mononuclear cells was determined by the above test, and the measured IC₅₀ values are shown in Table 8.

**Table 8**

| No. | DC IC₅₀ (nM) |
|---|---|
| Compound **I** | **0.018** |
| Roflumilast | **12.91** |
| Compound **A** | **0.61** |

### Test Example 4. Imiquimod-induced psoriasis suppression assay

An appropriate amount of Compound **I** was formulated into an ointment with the following components: 0.1% of Compound **I,** 9% of hexanediol, 78.8% of white petrolatum, 5% of paraffin, 7% of glyceryl mono- and di-stearate, and 0.1% of dihydroxybutyl toluene, which were mechanically stirred until an ointment was formed.

### 1) Model establishment and drug administration

7-Week-old Balb/c female mice were used and the hair on their back in an area of 2 cm × 3 cm was shaved the day before the experiment. On Days 1-7 of the experiment, the test compounds were applied on the skin, and after 6 hours, imiquimod (IMQ) ointment (Aldara (5%)) was continuously applied to the back skin of mice for 7 days, so as to establish a mouse model of psoriasis, while the control group was given the same dose of petrolatum ointment. On Days 3, 5 and 7, the severity of skin inflammation was assessed, including measurement of skin thickness, crusting and erythema, scored on a 5-point scale (0-5) respectively. The total score was used to assess the severity of skin inflammation. On Day 7 of the experiment, the spleen was collected and weighed, and the percentage of spleen to body weight was calculated to assess the immunosuppressive effect of the drug.

Normal control group, model control group, low, medium and high dose groups of Compound **I** (0.01%, 0.03% and 0.1%), and 0.03% reference Compound A group were set up in this experiment.

### 2) Assessment indicators

The total score of the degree of skin inflammation are the clinical score, the indicators of which are relatively subjective, and higher score indicated more serious disease. The degree of increase in skin thickness was an objective assessment indicator, and more increase in thickness indicated more serious disease. The ratio of spleen to body weight was an objective assessment indicator, and smaller ratio of spleen to body weight indicated stronger immunosuppressive effect of the drug.

### 3) Experimental results

### 3.1) Clinical scores

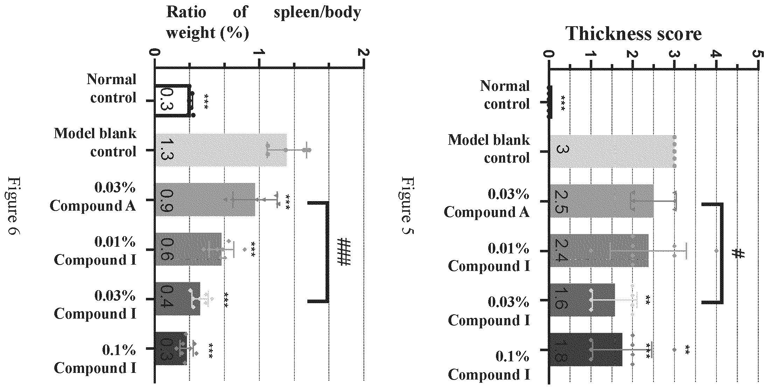

Compound **A** and each dose of Compound **I** significantly reduce the clinical score at the endpoint of the experiment (Figures 2-5). As seen from the scores of each single item, each dose group of Compound **I** mainly improves scab and reduces skin thickness in the psoriasis model. Among the skin thickness scores, both the low dose group and the high dose group of Compound **I** significantly inhibit the increase in skin thickness, while Compound **A** has no significant effect. 0.03% of Compound **I** has significantly higher inhibitory effect on the increase in skin thickness than Compound **A** at the same dose (Figure 5). The results show that Compound **I** is significantly better than the reference Compound A in improving psoriasis symptoms. The specific score data are shown in Table 9 below.

**Table 9**

| Group | Total clinical score | | Erythema score | | Psoriasisscore | | Thickness score | |
|---|---|---|---|---|---|---|---|---|
| | Mean | SD | Mean | SD | Mean | SD | Mean | SD |
| Normal control | 0 | 0 | 0 | 0 | 0 | 0 | 0 | 0 |
| Model blank control | 11 | 0 | 4 | 0 | 4 | 0 | 3 | 0 |
| 0.03% Compound **A** | 8.33 | 1.03 | 3.33 | 0.52 | 2.5 | 0.55 | 2.5 | 0.55 |
| 0.01% Compound **I** | 9.5 | 1.19 | 4 | 0 | 3.13 | 0.35 | 2.38 | 0.92 |
| 0.03% Compound **I** | 7.57 | 0.53 | 3.86 | 0.38 | 2.14 | 0.38 | 1.57 | 0.53 |
| 0.1% Compound **I** | 6.75 | 1.17 | 3.63 | 0.52 | 1.38 | 0.52 | 1.75 | 0.71 |

### 3.2) Ratio of spleen to body weight

Under the effect of IMQ, there is no significant difference in the body weight of animals between the model group and each dosing group, while the ratio of spleen to total body weight in the model group is increased significantly (Figure 6), indicating splenomegaly. Compound **A** has no significant effect on the ratio of spleen to body weight, while the three dose groups of Compound **I** significantly reduce the ratio of spleen to body weight, indicating that these compounds have immunosuppressive effect and Compound **I** shows a dose-response effect. At the same dose (0.03%), the effect of Compound **I** on the ratio of spleen to body weight is significantly different from that of Compound **A,** and these results indicate that the effect of Compound **I** on immunity is stronger than that of Compound **A.** The specific data are shown in Table 10 below.

**Table 10**

| | Normal control | Model blank control | 0.03% | 0.01% Compound **I** | 0.03% Compound **I** | 0.1% Compound **I** |
|---|---|---|---|---|---|---|
| | | | Compound **A** | | | |
| Mean | 0.34 | 1.26 | 0.96 | 0.64 | 0.43 | 0.31 |
| SD | 0.02 | 0.19 | 0.21 | 0.12 | 0.08 | 0.06 |

## Claims

1. Crystal form A of a compound of formula I, **characterized in that** the crystal form A has an X-ray powder diffraction spectrum represented by diffraction angle 2*θ* angle having characteristic peaks at 7.537°, 15.219°, 21.798° and 22.911°, preferably having characteristic peaks at 7.537°, 11.366°, 14.297°, 15.219°, 16.190°, 17.623°, 19.134°, 21.798°, 22.911° and 24.735°, more preferably having characteristic peaks at 7.537°, 11.366°, 14.018°, 14.297°, 15.219°, 16.190°, 17.623°, 19.134°, 21.062°, 21.798°, 22.911°, 24.735°, 25.123° and 26.884°, further preferably having characteristic peaks at 7.537°, 11.366°, 14.018°, 14.297°, 15.219°, 16.190°, 17.623°, 18.412°, 19.134°, 20.010°, 21.062°, 21.798°, 22.911°, 23.889°, 24.735°, 25.123°, 26.884°, 28.343°, 29.645°, 33.499° and 36.362°, and even further preferably the crystal form A has an X-ray powder diffraction spectrum represented by diffraction angle 2*θ* as shown in Figure 7,

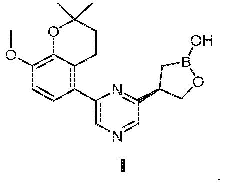

2. The crystal form A according to claim 1, wherein the error range of 2*θ* angle is ±0.2°.

3. A method for preparing the crystal form A according to claims 1 or 2, being method I or method II:
method I comprises the following steps of:
(a1) mixing the compound of formula I with a solvent (1);
(b1) crystallizing under stirring; 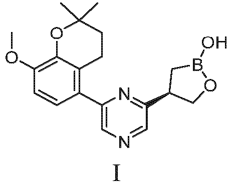
wherein, the solvent (1) is one or more selected from the group consisting of water, nitromethane, N,N-dimethylformamide, acetonitrile, methanol, ethanol, acetone, isopropanol, methyl ethyl ketone, tetrahydrofuran, 1,2-dimethoxyethane, chloroform, methyl tert-butyl ether, 1,2-xylene, toluene, dioxane, and hexane;
method II comprises the following steps of:
(a2) mixing the compound of formula I with a solvent (2);
(b2) crystallizing under volatilization;
wherein, the solvent (2) is one or more selected from the group consisting of acetone, acetonitrile, dichloromethane, dioxane, ethanol, ethyl acetate, heptane, isopropanol, methanol, methyl ethyl ketone, methyl tert-butyl ether, tetrahydrofuran, toluene, and water.

4. The method according to claim 3, wherein the solvent (1) is water, nitromethane, N,N-dimethylformamide, acetonitrile, ethanol, acetone, isopropanol, methyl ethyl ketone, tetrahydrofuran, 1,2-dimethoxyethane, chloroform, methyl tert-butyl ether, 1,2-xylene, toluene, dioxane, hexane, or a mixed solvent of water and methanol.

5. The method according to claim 4, wherein when the solvent (1) is a mixed solvent of water and methanol, and the molar ratio of the water to the methanol is 0.37 to 0.90, preferably 0.37, 0.40, 0.47, 0.50, 0.57, 0.60, 0.66, 0.70, 0.74, 0.80, 0.82, 0.90.

6. The method according to claim 3, wherein the solvent (2) is acetone, acetonitrile, dichloromethane, dioxane, ethyl acetate, heptane, isopropanol, methyl ethyl ketone, methyl tert-butyl ether, toluene, water, or any group of following mixed solvents: acetone and acetonitrile, acetone and dichloromethane, acetone and dioxane, acetone and ethanol, acetone and ethyl acetate, acetone and heptane, acetone and isopropanol, acetone and methanol, acetone and methyl ethyl ketone, acetone and methyl tert-butyl ether, acetone and tetrahydrofuran, acetone and toluene, acetone and water, acetonitrile and dichloromethane, acetonitrile and dioxane, acetonitrile and ethanol, acetonitrile and ethyl acetate, acetonitrile and isopropanol, acetonitrile and methyl ethyl ketone, acetonitrile and methyl tert-butyl ether, acetonitrile and tetrahydrofuran, acetonitrile and toluene, dichloromethane and dioxane, dichloromethane and ethanol, dichloromethane and ethyl acetate, dichloromethane and heptane, dichloromethane and isopropanol, dichloromethane and methyl ethyl ketone, dichloromethane and methyl tert-butyl ether, dichloromethane and tetrahydrofuran, dichloromethane and toluene, dioxane and methyl ethyl ketone, dioxane and tetrahydrofuran, dioxane and toluene, ethanol and ethyl acetate, ethanol and methanol, ethanol and tetrahydrofuran, ethanol and toluene, ethanol and water, ethyl acetate and heptane, ethyl acetate and isopropanol, ethyl acetate and methanol, ethyl acetate and methyl ethyl ketone, ethyl acetate and methyl tert-butyl ether, ethyl acetate and toluene, heptane and methyl ethyl ketone, heptane and methyl tert-butyl ether, heptane and tetrahydrofuran, heptane and toluene, methanol and methyl ethyl ketone, methanol and methyl tert-butyl ether, methanol and toluene, methanol and water, methyl ethyl ketone and methyl tert-butyl ether, methyl ethyl ketone and tetrahydrofuran, methyl ethyl ketone and toluene, methyl tert-butyl ether and tetrahydrofuran, methyl tert-butyl ether and toluene, tetrahydrofuran and toluene, tetrahydrofuran and water; preferably, when the solvent (2) is a mixed solvent, the volume ratio of the two is 1:3 to 3:1, preferably 1:1.

7. A pharmaceutical composition comprising the crystal form A according to claims 1 or 2 or the crystal form A prepared by the method according to any one of claims 3-6, and optionally a pharmaceutically acceptable excipient.

8. A method for preparing the pharmaceutical composition according to claim 7 comprising a step of mixing the crystal form A according to claims 1 or 2 or the crystal form A prepared by the method according to any one of claims 3-6 with a pharmaceutically acceptable excipient.

9. Use of the crystal form A according to claims 1 or 2 or the crystal form A prepared by the method according to any one of claims 3-6 or the pharmaceutical composition according to claim 7in the preparation of medicaments for the prevention and/or treatment of PDE-related diseases, preferably, the PDE-related disease is asthma, obstructive pulmonary disease, septicaemia, nephritis, diabetes, allergic rhinitis, allergic conjunctivitis, ulcerative enteritis or rheumatism.

10. Use of the crystal form A according to claims 1 or 2 or the crystal form A prepared by the method according to any one of claims 3-6 or the pharmaceutical composition according to claim 7 in the preparation of medicaments for the prevention and/or treatment of asthma, obstructive pulmonary disease, septicaemia, nephritis, diabetes, allergic rhinitis, allergic conjunctivitis, ulcerative enteritis or rheumatism.
